(19) Europäisches Patentamt
European Patent Office
Office européen des brevets

(11) EP 3 220 753 B1

# (12) EUROPEAN PATENT SPECIFICATION

(45) Date of publication and mention of the grant of the patent:
**17.07.2019 Bulletin 2019/29**

(21) Application number: **15831064.9**

(22) Date of filing: **19.11.2015**

(51) Int Cl.:
***A61K 31/661*** (2006.01)
***A23L 33/115*** (2016.01)
***A23L 33/12*** (2016.01)
***A61K 31/201*** (2006.01)
***A61K 31/202*** (2006.01)
***A61K 31/685*** (2006.01)
***A61P 25/00*** (2006.01)

(86) International application number:
**PCT/IB2015/002391**

(87) International publication number:
**WO 2016/079595 (26.05.2016 Gazette 2016/21)**

## (54) PHOSPHOLIPID PREPARATIONS FOR THE IMPROVEMENT OF COMMUNICATION SKILLS

PHOSPHOLIPIDZUBEREITUNGEN ZUR VERBESSERUNG DER KOMMUNIKATIONSFÄHIGKEITEN

PRÉPARATIONS DE PHOSPHOLIPIDES POUR L'AMÉLIORATION DES COMPÉTENCES DE COMMUNICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.11.2014 US 201462082261 P**

(43) Date of publication of application:
**27.09.2017 Bulletin 2017/39**

(73) Proprietor: **Enzymotec Ltd.**
**23106 Migdal HaEmeq (IL)**

(72) Inventors:
- **ZAAROOR REGEV, Daphna**
**3600100 Nofit (IL)**
- **CHUDNOW, Robert**
**Plano, TX 75093 (US)**
- **RICHTER, Yael**
**36589 (IL)**
- **SORIA ARTZI, Gali, Olga**
**18960 Haifa (IL)**

(74) Representative: **Kiriczi, Sven Bernhard**
**Schneiders & Behrendt PartmbB**
**Rechts- und Patentanwälte**
**Mühlthaler Strasse 91c**
**81475 München (DE)**

(56) References cited:
**WO-A1-2015/085192**
**WO-A2-2005/037848**
**US-A1- 2009 074 857**
**US-A1- 2013 289 277**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

## FIELD OF THE INVENTION

The present invention relates to phospholipid preparations effective in improving communication abilities in subjects suffering from communication impairments/difficulties.

## BACKGROUND OF THE INVENTION

Communication is giving, receiving or exchanging ideas, information, signals or messages, enabling individuals or groups to persuade, to seek information, to give information or to express emotions. This broad definition includes body-language, skills of speaking and writing.

Communication impairments/difficulties are developmental or acquired impairments. The American Speech-Language-Hearing Association (ASHA) describes communication disorders more specifically, as impacting one's ability to "receive, send, process, and comprehend concepts or verbal, nonverbal and graphic symbol systems." Communication impairments can partially or totally prevent communication.

Communication impairments may include, but are not limited to problems related to speech, language and auditory processing or social pragmatic communication. The last represent difficulties that influence the use of verbal and nonverbal communication in naturalistic contexts, which affects the development of social relationships and discourse comprehension.

Communication impairments are present in several disorders including, neurological disorders (e.g., epilepsy, aphasia, and brain injury/trauma); emotional or psychiatric disorders (e.g., generalized anxiety disorder, agoraphobia, social anxiety disorder, phobias, panic disorder, post-traumatic stress disorder, and selective mutism); personality disorders (e.g., schizophrenia and obsessive-compulsive disorder); developmental disorders (e.g., autism, Asperger syndrome, pervasive developmental disorder, childhood disintegrative disorder, Rett syndrome, and mental retardation); and speech disorders.

Autism spectrum disorder (ASD) covers a set of developmental disabilities that can cause significant social, communication, and behavioral challenges. ASD patients suffer from social communication disorder that is also characterized by restricted or repetitive pattern of behavior that causes significant functional impairment and distress for affected individuals and their caregivers. The terms Autism and ASD are herein interchangeably used.

ASD may be categorized on the basis of language development. Delays and/or abnormalities in language functioning are evident in some individuals with Autism while in others (such as Asperger patients) there is no clinically significant general delay in language although atypical language is often present as qualitative abnormalities such as: rate, volume, prosody, pragmatics, or frequency of speech production.

Another way to classify ASD is on the basis of communication patterns. Several studies have revealed that some individuals with autism (such as Asperger patients) have expressive/emotional communication patterns, whereas others use non-expressive/non-emotional communication patterns. Interestingly there is an association between the presentation of communication patterns and language development. Most ASD individuals without delay in language development present expressive/emotional communication patterns; whereas most ASD individuals suffering from a delay in language development also present non-expressive/non-emotional communication patterns.

US 2013/289277 A1 teaches a medicament comprising lurasidone or a pharmaceutically acceptable acid addition salt thereof for treating pervasive developmental disorders.

US 2009/074857 A1 teaches methods of improving cognitive functions in a subject using a lipid composition conjugated with omega-3 and omega-6 fatty acids, with specific amounts and specific conjugation patterns of LA, linolenic acid (alpha-linolenic acid, gamma-linolenic acid) DHA and eicosapentaenoyl (EPA), e.g. utilizing different sources of lipids. Disclosed herein is a lipid preparation, said preparation comprising a phosphatidylserine moiety, and poly-unsaturated fatty acid (PUFA) acyl groups, particularly long-chain poly-unsaturated fatty acid (LC-PUFA) acyl groups such as omega-3 and/or omega-6 acyl groups, wherein said PUFA is covalently bound to said glycerophospholipid. Said lipid preparations are particularly useful in the treatment of mental and cognitive disorders, e.g. ADHD (attention deficit hyperactivity disorder) and Alzheimer's disease

WO 2015/085192 A2 teaches a preparation for treatment and/or prevention of seizures comprising a non-mammalian derived mixture of serine glycerophospholipids (PS) conjugates, wherein the mixture comprises (a) Eicosapentaenoic acid (EPA) conjugated to PS and (b) Docosahexaenoic acid (DHA) conjugated to PS, and methods of treatment of seizures with same.

To the best of our knowledge, today there are no approved medications indicated to cure, prevent or manage communication disorders. Looking specifically at ASD, only 2 atypical antipsychotics, Risperidone and Aripiprazole, are approved by the US Food and Drug Administration (FDA) for treatment of significant irritability (aggression toward others, deliberate self-injuriousness, temper tantrums, and labile moods) associated with ASD. These medical interventions

benefit challenging behaviors however, they were not reported to improve verbal or non-verbal communication in ASD patients. Moreover, they have significant side effects including weight gain, sedation, and extrapyramidal effects.

Although medications are not correlated with a beneficial effect on communication patterns, there is increasing evidence that intervention approaches designed to enhance the communication and language development of children communication impairments can improve communication skills and abilities.

Glycerophospholipids, also referred to as phospholipids are key components of the lipid bilayer of cells, and are involved in cell metabolism and signaling. The hydroxyl groups of the glycerol backbone of phospholipids are substituted by a hydrophilic phosphate head and hydrophobic tail composed of non-polar fatty acids. Glycerophospholipids may be subdivided into distinct classes, based on the nature of the polar head group such as for example: phosphatidylcholine (also known as PC or lecithin), phosphatidylethanolamine (PE), and phosphatidylserine (PS). In addition to serving as a primary component of cellular membranes and binding sites for intracellular and intercellular proteins, some glycerophospholipids, such as phosphatidylinositols and phosphatidic acids are either precursors of, or are themselves, membrane-derived second messengers. Studies have shown that PS and PC enhance neuronal membrane function and improve memory skills. PS was found to have a beneficial effect in ADHD, depression, and chronic stress. In addition, PC was found to reduce emotional symptoms of premenstrual syndrome.

Apparently, the origin of the phospholipids and their fatty acid content influence their activity. For example, the bio-functionality of soybean PS in the improvement of cognitive function has been shown to be different from that of other types of PS (see, WO 2005/037848). In addition, it was demonstrated that different ratios of specific fatty acids conjugated to PS can influence the efficacy of the PS in improving cognitive functions in elderly subjects with impaired cognitive performance (see, WO 2009/156991).

It is thus beneficial to apply the appropriate type of PS preparation for the specific indication.

## SUMMARY OF THE INVENTION

The present invention provides a preparation comprising a phosphatidylserine (PS), a phosphatidic acid, a lysophosphatidic acid, and at least one fatty acid attached to the PS, wherein: a percentage by weight of PS with respect to the preparation is greater than 40%; a percentage by weight of PS with respect to total phospholipids in the preparation is greater than 40%; and a percentage of the at least one fatty acid attached to the PS in the preparation with respect to a total fatty acid content attached to the PS in the preparation is such that a percentage of Eicosapentaenoic acid (EPA) is greater or equal to a percentage of Palmitic acid, the percentage of Palmitic acid is greater than a percentage of Docosahexanoic acid (DHA), the percentage of DHA is greater than a percentage of Oleic acid, and the percentage of Oleic acid is greater than a percentage of Linoleic acid.

According to some embodiments, the percentage by weight of PS with respect to the preparation is at times greater than 45%, at times greater than 50%, and at times greater than 55%.

According to some embodiments, the percentage by weight of PS with respect to the total phospholipids in the preparation is at times greater than 45%, at times greater than 50%, at times greater than 55%, at times greater than 60%, and at times greater than 65%.

According to some embodiments, the ratio by weight of the PS to the sum of phosphatidic acid and lysophosphatidic acid in the preparation is greater than 1:1 and lower than 10:1, at times greater than 1.5:1 and lower than 8:1, at times greater than 2:1 and lower than 5:1, and at times greater than 2.5:1 and lower than 4:1.

According to some embodiments, the percentage of EPA attached to the PS in the preparation with respect to the total fatty acids content attached to the PS in the preparation is greater than 18% and lower than 45%, at times greater than 22% and lower than 40%, at times greater than 26% and lower than 36%, and at times greater than 27% and lower than 34%.

According to some embodiments, the percentage of Palmitic acid attached to the PS in the preparation with respect to the total fatty acids content attached to the PS in the preparation is greater than 18% and lower than 45%, at times greater than 22% and lower than 40%, at times greater than 26% and lower than 36%, and at times greater than 27% and lower than 34%.

According to some embodiments, the percentage of Palmitic acid attached to the PS in the preparation with respect to the total fatty acids content attached to the PS in the preparation is greater than 14% and lower than 42%, at times than 18% and lower than 40%, at times greater than 20% and lower than 30%, and at times greater than 21% and lower than 26%.

According to some embodiments, the percentage of DHA attached to the PS in the preparation with respect to the total fatty acids content attached to the PS in the preparation is greater than 6% and lower than 25%, at times greater than 8% and lower than 22%, at times greater than 11% and lower than 20%, and at times greater than 12% and lower than 17%.

According to some embodiments, the percentage of Oleic acid attached to the PS in the preparation with respect to the total fatty acids content attached to the PS in the preparation is greater than 1% and lower than 15%, at times

greater than 2% and lower than 13%, at times greater than 4% and lower than 11%, and at times greater than 5% and lower than 8%.

According to some embodiments, the percentage of Linoleic acid attached to the PS in the preparation with respect to the total fatty acids content attached to the PS in the preparation is greater than 0.1% and lower than 6%, at times greater than 0.5% and lower than 4%, at times greater than 1% and lower than 3%, at times greater than 1 and lower than 2, and at times greater than 1.5% and lower than 2%.

The present invention also provides a preparation according to any one of the above embodiments further comprising a phosphatidylcholine (PC). At times, the preparation has a percentage by weight of PC lower than 10% with respect to the preparation. At times, the percentage by weight of PC with respect to the preparation is greater than 0.01% and lower than 8%, at times greater than 0.01% and lower than 6%, at times greater than 0.01% and lower than 3.2%, at times greater than 0.05% and lower than 3.2%, at times greater than 0.1% and lower than 4%, and at times greater than 1% and lower than 3.5%.

According to a specific embodiment, the preparation of the invention comprises a phosphatidylserine (PS), a phosphatidic acid, a lysophosphatidic acid, a phosphatidylcholine (PC), and at least one fatty acid attached to the PS, wherein: a percentage by weight of PS with respect to the preparation is greater than 40%; a percentage by weight of PS with respect to total phospholipids in the preparation is greater than 50%; a percentage by weight of PC with respect to the preparation is greater than 0.01% and lower than 3.2%; a ratio by weight of the PS to the sum of the phosphatidic acid and the lysophosphatidic acid in the preparation is greater than 2.5:1 and lower than 4:1; a percentage of EPA attached to the PS in the preparation with respect to the total fatty acid content attached to the PS in the preparation is greater than 27% and lower than 34%; a percentage of Palmitic acid attached to the PS in the preparation with respect to the total fatty acids content attached to the PS in the preparation is greater than 21% and lower than 26%; a percentage of DHA attached to the PS in the preparation with respect to the total fatty acids content attached to the PS in the preparation is greater than 12% and lower than 17%; a percentage of Oleic acid attached to the PS in the preparation with respect to the total fatty acids content attached to the PS in the preparation is greater than 5% and lower than 8%; and a percentage of Linoleic acid attached to the PS in the preparation with respect to the total fatty acids content attached to the PS in the preparation is greater than 1% and lower than 2%.

The present invention provides a nutritional, pharmaceutical, or nutraceutical composition, or a functional or medical food comprising a preparation of the invention. In an embodiment, a pharmaceutical composition of the invention further comprises at least one pharmaceutically active agent.

According to one of its aspects the present invention provides a preparation or a composition according to the invention for use in a method for treating a communication disorder or impairment comprising administering to a subject in need thereof a therapeutically effective amount of any of the phospholipid preparations or compositions according to the invention. More preferably, the therapeutically effective amount is provided as a daily dose. Optionally, the method relates to treating and/or preventing the autistic spectrum disorder (ASD), autism, or Asperger syndrome. At times, the method relates to treating and/or preventing autism with language delay, low intellectual performance, non-expressive/non-emotional communication patterns, or a combination thereof; or autism without language delay and with normal/high IQ, and/or with expressive/emotional communication patterns.

## DETAILED DESCRIPTION OF THE INVENTION

As used herein treating communication disorder or impairment relates to improving the communication abilities of a patient with the communication disorder or impairment, reducing the severity or frequency of symptoms associated with the communication disorder or impairment, curing the communication disorder or impairment, improving the quality of life of a patient with the communication disorder or impairment, reducing the severity of comorbidities that accompany the communication disorder or impairment, or a combination thereof.

According to another embodiment of the invention, the phospholipid preparations may be administered to subjects with neurological diseases, preferably with neuro-developmental diseases, more preferably with diseases which are not related to age associated cognitive decline or neurodegeneration. According to another embodiment of the invention, the phospholipid preparations are administered to subjects at least one month old, at times, at least three years old, at times, at least six years old, and at times at least 13 years old. Optionally, the preparation and/or method according to at least some embodiments of the invention relates to improving communication in adults.

The use of the claimed compositions also relates to reducing the frequency of symptoms related to Autism, reducing the severity of symptoms related to Autism, ameliorating undesired symptoms associated with Autism, treating Autism, or a combination thereof by using the preparation of the invention. Preferably, the use of the claimed compositions relates to improving communication in communication impairments presented in Autism. At times, the use of the claimed compositions relates to reducing the frequency of symptoms related to Autism, reducing the severity of symptoms related to Autism, ameliorating undesired symptoms associated with Autism, treating Autism, or a combination thereof by using the preparation of the invention in autistic patients suffering from language delay, low intellectual performance, non-expressive/non-emotional communication patterns, or a combination thereof; or in autistic patients without language delay, with normal/high IQ, and/or with expressive/emotional communication patterns.

At times, the use of the claimed compositions relates to improving communication in communication impairments presented in autistic patients suffering from language delay, low intellectual performance, non-expressive/non-emotional communication patterns, or a combination thereof; or in autistic patients without language delay, with normal/high IQ, and/or with expressive/emotional communication patterns.

In another embodiment of the present invention the preparation is provided as a pharmaceutical composition in admixture with pharmaceutically acceptable auxiliaries, and optionally other therapeutic agents. The auxiliaries must be "acceptable" in the sense of being compatible with the other ingredients of the composition and not deleterious to the recipients thereof.

According to another embodiment, the pharmaceutical or nutraceutical compositions are in a dosage delivery form selected according to the route of administration.

According to another embodiment, the present invention can be administered in the form of capsules, tablets, pills, gummies, fluid oils, powders, granules, waxes, pastes, aqueous emulsions, and any other form that will enable its use in the target applications.

According to another embodiment, a daily dose of the preparation of the invention as described herein optionally provides 75-600 mg PS to the subject, at times 75-450 mg PS, at times 75-300 mg PS, at times 75-225 and at times 75-150 mg PS. The daily dose may optionally be divided to a plurality of doses each day or alternatively may optionally be delivered as a single bolus each day.

According to another embodiment, a daily dose of the preparation of the invention as described herein optionally provides 20-172 mg EPA to the subject, at times 21.5-129 mg EPA, at times 21.5-86 mg EPA and at times 21.5-43 mg EPA. The daily dose may optionally be divided to a plurality of doses each day or alternatively may optionally be delivered as a single bolus each day.

According to another embodiment, a daily dose of the preparation of the invention as described herein optionally provides 8-68 mg DHA to the subject, at times 8-51 mg DHA, at times 8-34 mg DHA and at times 8.5-17 mg DHA. The daily dose may optionally be divided to a plurality of doses each day or alternatively may optionally be delivered as a single bolus each day. The daily dose according to at least some embodiments of the present invention, when administrated as capsules, tablets, syrups, gummies, spray, syringe, dropper, tube' snorting (for powder), squeeze bottle delivery, atomized intranasal delivery (syringe or pump driven spraying devices), and other known delivery systems, optionally comprises one, two, three, four, five, six, seven or eight delivery units per day.

It should be noted that the preparation of the invention may also comprise other phospholipids, such as phosphatidylcholine (PC), phosphatidylethanolamine (PE), phosphatidylinositol (PI), and phosphatidylglycerol (PG), to which fatty acid acyls are covalently attached (bonded) at either or both of the *sn*-1 or *sn*-2 positions of the glycerol moiety of the phospholipid. The fatty acid conjugation profile of any of the above-noted polar lipids may be the same as, or different from, the fatty acid conjugation profile of PS, as disclosed herein.

The term "communication" is defined as any form of giving, receiving or exchanging ideas, information, signals or messages, enabling individuals or groups to persuade, to seek information, to give information or to express emotions. This broad definition includes but is not limited to body-language social interaction, eye contact, and skills of speaking and writing.

The terms "communication impairments" or "communication difficulties" or "communication disorders" are used herein interchangeably. The terms "communication impairments" or "communication difficulties" or "communication disorders" refer to disorders impacting one's ability to receive, send, process, and comprehend concepts or verbal, nonverbal and graphic symbol systems. Communication impairments can partially or totally diminish or prevent communication. Non-limiting examples for types of communication impairments are problems related to speech, language, auditory processing, or social pragmatic communication.

Non-limiting examples for disorders that may present communication impairments include: neurological disorders (e.g., epilepsy, aphasia , and brain injury or trauma); emotional or psychiatric disorders (e.g., generalized anxiety disorder, agoraphobia, social anxiety disorder, phobias, panic disorder, post-traumatic stress disorder, and selective mutism); personality disorders (e.g., schizophrenia and obsessive-compulsive disorder); neurodevelopmental disorders (e.g., autistic spectrum disorder (ASD), autism, Asperger syndrome, pervasive developmental disorder (PDD), childhood disintegrative disorder, Rett syndrome, and mental retardation); and speech disorders.

The term "improving communication" is described as reducing the frequency of symptoms related to communication impairments, reducing the severity of symptoms related to communication impairment, ameliorating undesired symptoms associated with communication impairment, reducing severity of a disease or disorder, curing a disease or disorder, preventing a disease or disorder from occurring altogether (for example, in an individual genetically and/or phenotypically prone to the disease), or a combination of any of the above. For example, in a subject suffering from communication impairment, improvement is expected by use of the lipid preparation of the invention.

Non-limiting examples of aspects of communication evaluated for improvement, development, or increase

include: verbal, nonverbal, and social communication. For example these aspects may include: vocabulary, pronunciation, vocalization, fluency of speech, reading level, listening skills, comprehension, ability to follow conversations, ability to take turns speaking, conversation skills, attention span, eye contact, facial expressions, hand gestures, body movements, social interaction, and social play.

The present invention also provides a nutritional, pharmaceutical, or nutraceutical composition or a functional or medical food comprising any one of the preparations mentioned above.

A nutritional composition as described herein can be any nutritional composition including, but not limited to: human milk fat substitute, infant formula, adult formula, dairy product, milk powder, ice-cream, biscuit, soy product, bakery, pastry, cake, bread, sauce, soup, instant food, prepared food, frozen food, condiment, confectionary, oil, fat, margarine, spread, filling, cereal, instant product, drink, shake, infant food, toddler food, bar, snack, candy, and chocolate product.

The term "infant formula" as used herein encompasses infant formulas (for newborn to 6 months old infants), follow-up formulas (for 6-12 months old babies), and growing up formulas (for 1-3 years old children).

A functional food as used herein can be any functional food, including, but not limited to: dairy product, ice-cream, biscuit, soy product, bakery, pastry, cake, bread, sauce, soup, instant food, prepared food, frozen food, condiment, confectionary, oil, fat, margarine, spread, filling, cereal, instant product, drink, shake, infant food, bar, snack, candy, and chocolate product.

A nutraceutical composition as used herein can be any nutraceutical, which can be any substance that may be considered a food or part of a food and provides medical or health benefits, including the prevention and treatment of diseases or disorders. Such nutraceutical compositions include, but are not limited to: a food additive, a food supplement, phospholipid mixtures, PS or PC from other sources, a dietary supplement, genetically engineered foods (such as for example vegetables), herbal products, processed foods (such as cereals, soups and beverages), stimulant functional food, clinical nutrition product, medical food and pharma food. Dietary supplements may be delivered in the form of soft gel capsules, tablets, syrups, gummies, candies, and other known dietary supplement delivery systems.

A medical food as used herein is specially formulated and intended for the dietary management of a disease/disorder that has distinctive nutritional needs that cannot be met by normal diet alone.

Suitable routes of administration for the compositions of the subject invention are oral, nasal, intranasal, inhalation, buccal, sublingual administration, administration via a feeding tube, topical, transdermal, or parenteral (including subcutaneous, intramuscular, intravenous and intradermal) administration. In an embodiment, the compounds are administered orally.

The pharmaceutical, nutraceutical or medical food compositions may be in any of the many dosage delivery forms commonly used in the art. Pharmaceutical, nutraceutical or medical food compositions suitable for oral administration may be presented as discrete dosage units (such as pills, tablets, pellets, dragees, capsules, or softgel), as a powder or granule, or as a solution, suspension, syrup, or elixir.

The present invention also provides pharmaceutical compositions wherein a preparation according to the invention is admixed with (pharmaceutically) acceptable auxiliaries, and optionally other therapeutic agents. The auxiliaries must be "acceptable" in the sense of being compatible with the other ingredients of the composition and not deleterious to the recipients thereof.

In one embodiment of the present invention, a pharmaceutical composition of the present invention further comprises at least one additional pharmaceutically active agent.

For parenteral administration, suitable compositions include aqueous and non-aqueous sterile injection. The compositions may be presented in unit-dose or multi-dose containers, for example sealed vials and ampoules, and may be stored in a freeze-dried (lyophilized) condition requiring only the addition of sterile liquid carrier, for example water, prior to use. For transdermal administration, e.g. gels, patches or sprays can be contemplated.

The compositions may be presented in unit-dose or multi-dose containers, for example sealed vials and ampoules, and may be stored in a freeze-dried (lyophilized) condition requiring only the addition of sterile liquid carrier, for example water, prior to use.

Compositions suitable for oral administration may be presented as discrete dosage units such as pills, tablets, pellets, dragees, capsules, powders, granules, solutions, suspensions, or elixirs.

The exact dose and regimen of administration of the composition will necessarily be dependent upon the therapeutic effect to be achieved and may vary with the particular formula, the route of administration, and the age and condition of the individual subject to whom the composition is to be administered.

The pharmaceutical and nutraceutical compositions of the present invention may be prepared by any method well known in the art of pharmacy. Such methods include the step of bringing in association the ingredients with any auxiliary agent. The auxiliary agent(s), also named accessory ingredient(s), include those conventional in the art, such as carriers, fillers, binders, diluents, desiccants, disintegrants, lubricants, colorants, flavoring agents, anti-oxidants, and wetting agents.

The pharmaceutical and nutraceutical compositions of the invention may further comprise edible fibers, aroma,

taste ingredients, and ingredients that control physical and organoleptic properties.

The terms *"glycerophospholipid"* and *"phospholipids"* are used herein interchangeably and should be understood to encompass a lipid of the general formula:

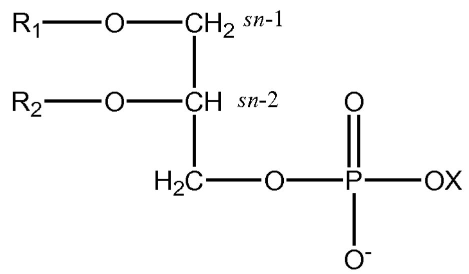

Wherein X represents a moiety selected from serine, choline, ethanolamine, inositol, glycerol and hydrogen, and R1 and R2, which may be identical or different, independently represent hydrogen or an acyl group, wherein the acyl group is selected from saturated, mono-unsaturated or poly-unsaturated acyl groups (PUFA). The *sn*-1 and *sn*-2 positions as used herein and as indicated in above formula, refer to the respective carbon atoms on the glycerol backbone wherein $R_1$ and $R_2$, are hydrogen or acyl groups substituted on the corresponding position.

The term "lysophosphatidic acid" is used herein when X represents hydrogen and one of R1 or R2 is Hydrogen as well.

As described herein, the terms *"substituted," "conjugated,",* and *"attached"* are used interchangeably and should be understood to encompass a fatty acid acyl covalently attached to the glycerophospholipid backbone of a phospholipid of the invention. As noted above, the fatty acid may be attached to the *sn*-1 and/or *sn*-2 positions.

As used herein, the term *"fatty acid"* should be understood to encompass a carboxylic acid with a long unbranched aliphatic tail (chain), which is either saturated, or unsaturated having one unsaturated bond (mono-unsaturated fatty acids) or two or more unsaturated bonds (poly-unsaturated fatty acids). When referring to a *"fatty acid acyl"* it should be understood to encompass an -C(=O)-R radical wherein R is a long unbranched aliphatic tail, which is either saturated or unsaturated having one unsaturated bond (mono-unsaturated fatty acids) or two or more unsaturated bonds (poly-unsaturated fatty acids).

As used herein, the term ω-X, Omega-X, n-X (X denotes a number), are interchangeably used and should be understood to denote the carbon atom furthest from the carboxyl group of a fatty acid.

Non-limiting examples of saturated fatty acids include: Butyric acid (Butanoic acid, C4:0), Caproic acid (Hexanoic acid, C6:0), Caprylic acid (Octanoic acid, C8:0), Capric acid (Decanoic acid, C10:0), Lauric acid (Dodecanoic acid, C12:0), Myristic acid (Tetradecanoic acid, C14:0), Palmitic acid (Hexadecanoic acid, C16:0), Stearic acid (Octadecanoic acid, C18:0), Arachidic acid (Eicosanoic acid, C20:0), Behenic acid (Docosanoic acid C22:0).

Non-limiting examples of unsaturated fatty acids include: Myristoleic acid (C14:1, ω-5), Palmitoleic acid (C16:1, ω-7), Oleic acid (C18:1, ω-9), Linoleic acid (C18:2, ω-6), Linolenic acid (C18:3) [Alpha-linolenic acid (C18:3, ω-3), Gamma-linolenic acid (C18:3, ω-6)], Eicosenoic acid (C20:1, ω-9), Arachidonic acid (C20:4, ω-6), Eicosapentaenoic acid (C20:5, ω-3), Erucic acid (C22:1, ω-9), Docosapentanoic acid (C22:5, ω-3) and Docosahexaenoic acid (C22:6, ω-3), Nervonic acid (C24:1, ω-9).

The term a *"[fatty acid] conjugated to phospholipid,"* should be understood to encompass a phospholipid wherein a fatty acid acyl is conjugated at position *sn*-1 and/or position *sn*-2 of the phospholipid backbone (through the glycerol oxygen atom). In one embodiment, a fatty acid is conjugated at position *sn*-1, and position *sn*-2 is either unsubstituted (e.g. having a hydrogen atom on the glycerol oxygen) or substituted with an acyl group selected from saturated, mono-unsaturated and polyunsaturated fatty acids, which may be the same or different from the substitution on position *sn-1.* In another embodiment, a fatty acid is conjugated at position *sn*-2 and position *sn*-1 is either unsubstituted (e.g. having a hydrogen atom on the glycerol oxygen) or substituted with an acyl group selected from saturated, mono-unsaturated and polyunsaturated fatty acids, which may be the same or different from the substitution on position *sn*-2.

The term phosphatidylserine is often also referred to in the literature as serine glycerophospholipid, phosphatidyl serine, and PS.

The term phosphatidylcholine is often also referred to in the literature as Choline glycerophospholipid, phosphatidyl choline, and PC.

A preparation of the invention may also be administered in conjunction with other compounds, including, but not limited to folic acid, vitamins, minerals, amino acids, nucleotides, antioxidants and so forth.

It will be appreciated that a composition (whether pharmaceutical, nutraceutical, nutritional, medical food, etc.) or product (e.g. functional food) of the invention may be combined with other treatment methods known in the art. Thus, treatment of communication impairment (e.g. Autism) using a composition or product of the invention may optionally be

combined with conventional therapies for communication impairment such as but not limited to: stimulants, anti-stimulants, methylphenidate, dextroamphetamine clonidine, guanfacine, risperidone, haloperidol, olanzapine, thioridazine, fluoxetine, sertraline, lithium, lorazepam carbamazepine, valproic acid.

Disclosed and described, it is to be understood that this invention is not limited to the particular examples, process steps, and materials disclosed herein as such process steps and materials may vary somewhat. It is also to be understood that the terminology used herein is used for the purpose of describing particular embodiments only and not intended to be limiting since the scope of the present invention will be limited only by the appended claims and equivalents thereof.

It must be noted that, as used in this specification and the appended claims, the singular forms "a", "an" and "the" include plural referents unless the content clearly dictates otherwise.

Throughout this specification and the claims which follow, unless the context requires otherwise, the word *"comprise",* and variations such as *"comprises"* and *"comprising,"* will be understood to imply the inclusion of a stated integer or step or group of integers or steps but not the exclusion of any other integer or step or group of integers or steps.

A preparation as described herein may be optionally prepared through enzymatic, chemical or molecular biology methods. Briefly, a phospholipid mixture can be enriched with the required fatty acids (e.g. EPA, Palmitic acid, DHA, oleic acid, linoleic acid) by enzymatic processes, e.g. enrichment of a natural phospholipid with specific fatty acids by enzymatic transesterification/esterification. Another pathway to acquire the preparation is to obtain a phospholipid source which is naturally rich in the required fatty acids, such as marine-derived lecithin (e.g. krill, fish, algae, and squid) or eggs phospholipids. Usually, In order to obtain the requested ratio between the different phospholipids in the mixture transformation of the phospholipid head group to serine (using PLD enzymes) is required to obtain PS. Such methods have been described in WO 2005/038037. Alternatively, the phospholipid mixture, according to at least some embodiments of the present invention can be prepared by GMO (genetically modified organisms)/biotechnology methods, for example, providing phospholipids-producing organisms with the required fatty acids to obtain the different phospholipids conjugates.

According to another embodiment of the present invention, the preparation is preferably prepared from a natural, synthetic or semi-synthetic source or any combinations thereof. In an embodiment of the present invention, the natural source is derived from any one of plant (such as for example soy and algae), non-mammalian animal (such as for example krill, fish (such as for example Herring and blue Whiting), or microorganism (such as for example bacteria) source or any combinations thereof. In yet a further embodiment, the production of the lipid preparation involves an enzymatic catalysis.

**Quantification of Phospholipids by 31P-NMR spectroscopy using the internal standard method.**

Purpose: This method is used to determine the phospholipid content by weight in the preparation.

Instruments: Bruker Avance III 600 MHz with automatic sample changer and cQNP probe head. Bruker Avance 300 MHz with automatic sample changer and BBI probe head. For the quantification of phospholipids in the preparation of the invention (powder form) approximately 300 mg of the test substance and 20 mg of internal standard TPP (triphenylphosphate) is dissolved in 1.5 ml CDCl3, 3 ml methanol and 3 ml aqueous Cs-EDTA solution (0.2 m, pH 7.5). After 15 minutes of shaking, the organic layer is separated by centrifugation and measured with 31P-NMR. The integrated signals of the test substance and of the internal standard TPP (triphenylphosphate) are used for calculation. The ratio of integrals corresponds to the molar ratio of the compared substances. For calculation software Microsoft Excel 14.0 is used.

Calculation:

| | |
|---|---|
| Equation 1 | $MOL_{IS}\,[mMol] = \dfrac{W_{IS}\,[mg] * C_{IS}\,[\%]}{MW_{IS}\,[g/Mol] * 100}$ |
| Equation 2 | $MOL_{P}\,[mMol] = \dfrac{I_{P} * H_{IS} * MOL_{IS}\,[mMol]}{I_{IS} * H_{P}}$ |
| Equation 3 | $weight\text{-}\%_{P} = \dfrac{MW_{P}\,[g/Mol] * MOL_{P}\,[mMol] * 100}{W_{P}\,[mg]}$ |

Declaration of variables:

| | test substance | internal standard |
|---|---|---|
| molecular weight | $MW_P$ (According to the MW table presented below) | $MW_{IS}$ |
| initial weight [mg] | $W_P$ | $W_{IS}$ |
| content [%-by weight] | weight-$\%_P$ | $C_{IS}$ |
| Mol [mMol] | $MOL_P$ | $MOL_{IS}$ |
| integral | $I_P$ | $I_{IS}$ |
| number of P-atoms | $H_P$ | $H_{IS}$ |

| **Phospholipid** | **$MW_P$ (g/mol)** |
|---|---|
| Phosphatidylcholine (PC) | 812.0 |
| Lyso Phosphatidylcholine (LPC) | 534.5 |
| Phosphatidylinositol (PI) | 907.0 |
| Lyso Phosphatidylinositol (LPI) | 629.5 |
| Phosphatidylserine (PS) | 833.0 |
| Lyso Phosphatidylserine (LPS) | 555.5 |
| Phosphatidyl Ethanolamine (PE) | 770.0 |
| Lyso Phosphatidyl Ethanolamine (LPE) | 492.5 |
| Phosphatidic Acid (PA) | 746.0 |
| Lyso Phosphatidic Acid (LPA) | 468.5 |
| Acyl Phosphatidyl Ethanolamine (APE) | 1032.0 |
| Other | 812.0 |

**Determination of Fatty Acid Percentage in Phospholipids**

Purpose: This method is used to determine the percentage of a fatty acid attached to the PS in the preparation with respect to the total fatty acid content attached to the PS in the preparation.

Materials: Acetic acid glacial A.R., Methanol abs. A.R., Chloroform A.R., Acetone A.R., Hexane A.R., Toluene A.R., Di-isopropyl ether AR., Butylhydroxytoluene, Sigma Lot#W218405 or equivalent, Sodium Sulfate Anhydrous, Sigma, Lot#31481, or equivalent, Sodium methoxide 25% (w/w) in methanol, Sigma Cat#15625-6, or equivalent, Primuline, Sigma Cat#206865, or equivalent, GC reference standard, Nuchek Lot#566B, Phosphatidylcholine reference standard, Sigma Aldrich Lot Cat#P3556, or equivalent, Phosphatidylserine reference standard, Sigma Aldrich Lot Cat#P5660, or equivalent, TLC Plates 20X10, silica gel 60 F254 layer MERCK 1.05715, or equivalent.

Apparatus: Orbital shaker with temperature control, Analytical Balance, Pipettor 0.2-1 ml and 1-5 ml range, Volumetric pipette 10ml class A,TLC tank, suitable for 20x10 TLC plates, Disposable capillaries 5$\mu$l volume, GC systems suitable for use with capillary column, equipped with oven capable of maintaining temperature with +0.1C degree accuracy, FID detector, split mode injection unit with temperature controller, GC capillary column, G16 USP phase, length 30m, I.D. 0.25mm, film 0.25$\mu$m, or similar.

Reagents and solutions preparation:

Sodium Methoxide solution: Accurately weigh 54 g of Sodium methoxide 25% into a 500 ml volumetric flask. Dilute to volume with Methanol Abs. Store in a dark place, in a tightly closed glass container. Solution is stable for up to 3 months.

Chloroform:Methanol 95:5 solution: Mix 95 volumes of Chloroform with 5 volumes of Methanol. Store in a dark place, in a tightly closed glass container. Solution is stable for up to one year.

Developing solution: Mix Water, Methanol, Acetic acid, Acetone and Chloroform in a volume ratio of 5:10:15:20:50, respectively. Store in a dark place, in a tightly closed glass container. Solution is stable for up to one year.

Primuline solution: Weight 10 mg into a 100 ml volumetric flask. Add 60 ml Acetone and 40 ml water. Mix well. Store in a dark place, in a tightly closed glass container. Solution is stable for up to one year.

Antioxidant solution 1 mg/ml: Weighed 25±2 mg Butylhydroxytoluene into a 25 ml volumetric flask. Add Toluene to the Mark, mix well (This solution can be kept for 3 month at room temperature.).

Antioxidant solution 0.05 mg/ml: Pipette 10 ml of the above solution into a 200 ml volumetric flask, add Toluene to the Mark, mix well. Store at 50°C for up to 3 months. (This solution can be kept for 3 months at room temperature).

PS/PC mix standard solution: Add about 20 mg of Phosphatidylserine reference standard into a 2 ml volumetric flask, add about 20 mg of Phosphatidylcholine reference standard. Add a small amount of Chloroform:Methanol solution sufficient to dissolve the reference standards. Once dissolved fill up to volume with the same Chloroform:Methanol solution. Store in a tightly closed container at -20°C. Stable for up to 3 months.

System suitability solution: Empty an ampoule containing 100 mg of GC reference standard 566B into a 50 ml volumetric flask, add 0.05 mg/ml Antioxidant Solution to the Mark. Mix well. Store in tightly closed container at -20°C. Stable for up to 3 months.

Procedure:

Sample solution preparation: Accurately weight 500 mg of the sample into a 20 ml vial with ground stopper. Add 10 ml Chloroform: Methanol solution and shake vigorously for 2-3 minutes.

Phospholipids purification: Perform test in duplicate. Perform blank determination by developing an unloaded plate (no sample applied to the plate). Sample silica from an area corresponding to the area of the sample followed by methylation as described above. Apply an even thin band of 120µl sample solution on TLC plate, 1cm above the plate bottom, leaving a 3cm margin on each side. At one of the margins, apply PS/PC mix standard solution of approximately 5µl, spot wise by means of a disposable capillary. Add 45 ml of di-isopropyl ether to the 20X10 mm Glass TLC chamber. Saturate the chamber for 15-20 minutes. Develop TLC plate up to about 90 mm mark. Dry the plate in fume hood under air at room temperature for about 10 minutes. Repeat the previous two steps once more using the same chamber. Add 45 ml of developing solution to the 20X10 mm Glass TLC chamber. Saturate the chamber for 15-20 minutes. Develop TLC plate up to about 80 mm mark. Dry the plate in fume hood under a current of air at room temperature for about 10 minutes. Spray the TLC plate evenly with Primuline solution and dry under a current of air at room temperature for about 10 minutes. Place the plate under UV lamp at 365 nm to observe the bands. Identify the corresponding bands using spots of PS mix reference standard and scrub the bands in-to a 20ml glass vial with ground stopper.

Methylation: To the 20 ml vials containing scrubbed silica add 2 ml Toluene. Then add 4 ml of Sodium methoxide solution. Shake for 15 minutes at 50°C. Then add 200 µl of Acetic acid and 4 ml of purified water, shake vigorously for 1 minute. Add 2 ml of Hexane and shake vigorously for 30 seconds. Transfer only the upper organic layer to a 20 ml bottle. Again add 2 ml of Hexane and shake vigorously for 30 seconds. Transfer only the upper organic layer to the same 20 ml bottle. Combine organic phases and dry over 0.5 grams Sodium sulfate. Filter through a 0.2 micron filter. Evaporate hexane under a nitrogen stream, until a volume of about 0.5 ml is reached. Analyze the sample by Gas Chromatography.

Gas Chromatography settings:

| | | | | | |
|---|---|---|---|---|---|
| Column | Capillary column, G16 USP phase, length 30m, I.D. 0.25mm, film 0.25µm, or similar | | | | |
| Carrier gas | Helium | | | | |
| Equilibration time | 2 min | | | | |
| Temperatures | Initial Temp | Initial Time | First Temp. rate | Final Temp. | Hold Time |
| | 170°C | 2 min | 1°C/min | 210°C | 2 min |
| | | | Second Temp. rate | Final Temp. | Hold Time |
| | | | 30°C/min | 240°C | 11 min |
| Injector temp. | 250°C | | | | |
| Pressure | 21 psi | | | | |
| Split ratio | 25:1 | | | | |
| Helium flow | 1.5 ml/min (constant flow) | | | | |
| Total flow | 41.4 ml/min | | | | |
| Detector temp. | 270°C | | | | |

(continued)

| | |
|---|---|
| Column | Capillary column, G16 USP phase, length 30m, I.D. 0.25mm, film 0.25μm, or similar |
| Hydrogen flow | 40 ml/min |
| Air flow | 400 ml/min |
| Injection volume | 1 μl |
| *Note:* Gas flow and temperature ramp may be adjusted to meet system suitability acceptance criteria. | |

Chromatography Injection Order: First inject Hexane and insure that there is no response in the relevant retention time. Next, inject System Suitability solution. The acceptance criteria is as follows: the resolution (R) between the peaks due to methyl oleate (C18:1n9) and methyl cis-vaccinate (C:181n11) ≥ 1.3.

$$resolution \quad R = \frac{2(t2 - t1)}{1.7(W1 + W2)}$$

where, t1 and t2 are the retention times of the two components and W1 and W2 are the corresponding widths at half-height of the peaks.

Next, inject sample from blank TLC plate (TLC blank). If there are peaks observed in the TLC blank chromatogram (except the solvent peak), they must be subtracted from the chromatogram of the sample. Finally, inject Samples.

Calculation: Calculate the area percentage of a fatty acid component in sample by the formula: % FA = AreaFA / AreaTot, where AreaFA is the area of the peak response obtained for an individual fatty acid methyl ester and AreaTot is the sum of the peak areas of all of the peaks, corresponding to fatty acids methyl esters. Report the results indicating two digits after decimal point. Relative standard deviation between the replicates should not exceed 5%.

The following Example is a representative of techniques employed by the inventors in carrying out aspects of the present invention. It should be appreciated that these techniques are exemplary of preferred embodiments for the practice of the invention.

**Example: The effect of the preparation of the invention in autistic patients**

Preparation of phospholipid composition: Lipid preparations were prepared as follows: marine lecithin produced by an extraction process from biomass derived from krill was dissolved in organic solvents and allowed to react with an aqueous solution containing L-serine, CaCl2, phospholipase D (PLD), and acetate buffer. Following the trans-phosphatidylation reaction, the resulting mixture that contained PS, PC, PA, and LPA was purified by removal of the water phase, evaporation of the organic solvents, and further purification stages. The resulting powder mainly contained EPA, Palmitic acid, DHA, oleic acid, and linoleic acid bound to the different phospholipids.

Table 1- list of ingredients in one capsule (167 mg per capsule) containing the phospholipid preparation

| *Parameter* | *Specification* |
|---|---|
| Phosphatidylserine | n.l.t. 75 *mg/capsule* |
| Phosphatidylcholine | n.l.t. 0.0165 *mg/capsule* |
| Phosphatidic acid | n.l.t.13 *mg/capsule* |
| Lysophosphatidic acid | n.l.t.2 *mg/capsule* |
| Total Phospholipids | n.l.t 130 mg/capsule |
| | |
| Total DHA | n.l.t. 8.5 *mg/capsule* |
| Total EPA | n.l.t. 21.5 *mg/capsule* |
| Total Palmitic acid | n.l.t. 16.5 *mg/capsule* |
| Total Oleic acid | n.l.t. 3.5 *mg/capsule* |
| Total Linoleic acid | n.l.t. 1 *mg/capsule* |

(continued)

| *Parameter* | *Specification* |
|---|---|
| Percentage by weight of phosphatidylserine (PS) with respect to the preparation | 40-48 % |
| Percentage by weight of PS with respect to total phospholipids in the preparation | 51-60 % |
| Percentage by weight of phosphatidylcholine (PC) with respect to the preparation | 0.01-3.2% |
| Ratio by weight of the PS to the sum of phosphatidic acid and lysophosphatidic acid | 2.5:1 - 4:1 |
| Percentage of EPA attached to the PS with respect to the total fatty acid content attached to the PS | 27-34% |
| Percentage of Palmitic acid attached to the PS with respect to the total fatty acid content attached to the PS | 21-26% |
| Percentage of DHA attached to the PS with respect to the total fatty acid content attached to the PS | 12-17% |
| Percentage of Oleic acid attached to the PS with respect to the total fatty acid content attached to the PS | 5-8 % |
| Percentage of Linoleic acid attached to the PS with respect to the total fatty acid content attached to the PS | 1-2% |
| *n.l.t- not less than* | |

Study design: The effect of the phospholipid composition on autistic patients was evaluated in 19 children (3 females and 16 males), 2-17 years of age, who were diagnosed with autism. All study patients received a daily dose of 1-4 capsules of the phospholipid composition for at least 3 months, with or without concomitant medication for the management of Autism.

The effect on the autistic symptoms was evaluated via electronic health record reviews and is described in Table 2. Of the study patients, 6 were classified as suffering from Autism without language delay and with expressive/emotional communication patterns (subclass 1). 13 were classified as suffering from delay in language development and non-expressive/ non-emotional communication patterns (subclass 2). All the participants received the phospholipid preparation. The dosage and the duration of the administration are described in table 2.

Results: In general, as demonstrated in Table 2, 10 out of the 19 patients evaluated in the study demonstrated an improvement in autistic symptoms during the study period (e.g. patient No. 4: "general improvement", patient No 12: "improved speech"). Interestingly, all the improvements were observed in the group of patients who suffered from language delay and had non-expressive/non-emotional communication patterns (subclass 2). In this group, 10 patients (No. 2, 3, 4, 5, 8, 9, 10, 11, 12, 13) demonstrated an improvement in autistic symptoms during the study period. In the group without language delay and with expressive/emotional communication patterns (subclass 1; 6 patients) no significant improvements were detected (No. 14, 15, 16, 17, 18, 19).

Of the patients who improved, 6 patients (No. 2, 5, 8, 9, 10, 12) demonstrated an improvement in their communication abilities: patient No. 2 made progress in speech, patient No. 5 talked more, patient No. 8 followed conversation well, patient No. 9 improved his social play, patient No. 10 became more conversational, and patient No. 12 improved his speech.

Table 2: The effect of Phospholipid mixture preparation on Autistic symptoms

| Serial No. | Serial no. in the Database | Treatment Duration (Months) | Amount of capsules per day | Age and gender | The preparation effect in managing Autistic symptoms | Autism subclass |
|---|---|---|---|---|---|---|
| 1 | 26 | 4.5 | 2 | 16M | No improvement | 2 |
| 2 | 34 | 6 | 2 | 2M | Improvement in communication skills: Making progress in speech. More interactive | 2 |
| 3 | 96 | 11 | 2 | 14M | General Improvement | 2 |

(continued)

| Serial No. | Serial no. in the Database | Treatment Duration (Months) | Amount of capsules per day | Age and gender | The preparation effect in managing Autistic symptoms | Autism subclass |
|---|---|---|---|---|---|---|
| 4 | 172 | 8 | 2 | 17M | General Improvement | 2 |
| 5 | 261 | 2 | 2 | 3F | Improvement in communication skills: Talking more | 2 |
| 6 | 266 | 3 | 2 | 9M | No improvement | 2 |
| 7 | 305 | 3 | 2 | 10M | No improvement | 2 |
| 8 | 367 | 3 | 2 | 13M | Improvement in communication skills: Follows conversations well, more clarity of thought | 2 |
| 9 | 377 | 4 | 2 | 6M | Improvement in communication skills: Improving social play | 2 |
| 10 | 414 | 3 | 2 | 13F | Improvement in communication skills: not looping, more conversational | 2 |
| 11 | 484 | 1.5 | 2 | 11F | General improvement | 2 |
| 12 | 512 | 3.5 | 1 | 6M | Improvement in communication skills: Improved speech | 2 |
| 13 | 646 | 3 | 2 | 9M | General Improvement | 2 |
| 14 | 168 | 6 | 2 | 11M | No improvement | 1 |
| 15 | 171 | 5 | 2 | 6M | No improvement | 1 |
| 16 | 253 | 5 | 2.4.2* | 8M | No improvement | 1 |
| 17 | 530 | 5 | 2 | 17M | No improvement | 1 |
| 18 | 581 | 19 | 2 | 16M | No improvement | 1 |
| 19 | 655 | 13 | 2 | 10M | No improvement | 1 |

Abbreviations: M-Male; F-Female
Subclass 1- Autism without language delay and with expressive/emotional communication patterns.
Subclass 2- Autism with a delay in language development and non-expressive/ non-emotional communication patterns.
*2.4.2. Started from 2 caps per day, increased to 4 and then reduced to 2.

**Claims**

**1.** A preparation comprising:

a phosphatidylserine (PS),
a phosphatidic acid,
a lysophosphatidic acid, and
at least one fatty acid attached to the PS, wherein:

a percentage by weight of the PS with respect to the preparation is greater than 40%;
a percentage by weight of PS with respect to total phospholipids in the preparation is greater than 40%;
and a percentage of the at least one fatty acid attached to the PS with respect to a total fatty acid content attached to the PS in the preparation is such that the percentage of Eicosapentaenoic acid (EPA) is greater or equal to the percentage of Palmitic acid, the percentage of Palmitic acid is greater than the percentage of Docosahexanoic acid (DHA), the percentage of DHA is greater than the percentage of Oleic acid, and

the percentage of Oleic acid is greater than the percentage of Linoleic acid.

2. The preparation of any one of the preceding claims, wherein the percentage by weight of PS with respect to the total phospholipids in the preparation is greater than 50%.

3. The preparation of any of the preceding claims, wherein the percentage of EPA attached to the PS in the preparation with respect to the total fatty acid content attached to the PS in the preparation is greater than 18% and lower than 45%, optionally greater than 27% and lower than 34%.

4. The preparation of any one of the preceding claims, wherein the percentage of Palmitic acid attached to the PS in the preparation with respect to the total fatty acids content attached to the PS in the preparation is greater than 14% and lower than 42%, optionally greater than 21% and lower than 26%.

5. The preparation of any one of the preceding claims, wherein the percentage of DHA attached to the PS in the preparation with respect to the total fatty acids content attached to the PS in the preparation is greater than 6% and lower than 25%, optionally greater than 12% and lower than 17%.

6. The preparation of any one of the preceding claims, wherein the percentage of Oleic acid attached to the PS in the preparation with respect to the total fatty acids content attached to the PS in the preparation is greater than 1% and lower than 15%, optionally greater than 5% and lower than 8%.

7. The preparation of any one of the preceding claims, wherein the percentage of Linoleic acid attached to the PS in the preparation with respect to the total fatty acids content attached to the PS in the preparation is greater than 0.1% and lower than 6.0%, optionally greater than 1% and lower than 2%.

8. The preparation of any one of the preceding claims, further comprising a phosphatidylcholine (PC).

9. The preparation of claim 8, wherein a percentage by weight of the PC with respect to the preparation is less than 10.0%, optionally greater than 0.01 and lower than 3.2%.

10. The preparation of any one of the preceding claims, wherein a ratio by weight of the PS to the sum of phosphatidic acid and the lysophosphatidic acid in the preparation is greater than 1:1 and lower than 10:1, optionally greater than 2.5:1 and lower than 4:1.

11. A composition comprising the preparation of any one of the above claims wherein the composition is a nutritional composition, nutraceutical, functional food, or medicinal food.

12. A pharmaceutical composition comprising the preparation of any one of the above claims.

13. The pharmaceutical composition of claim 12, further comprising at least one pharmaceutically active agent.

14. The preparation of any one of claims 1-10 or the composition of claims 11, 12 or 13 for use in the treatment of communication impairment associated with autistic spectrum disorder (ASD), autism, or Asperger syndrome.

**Patentansprüche**

1. Präparat, umfassend:

ein Phosphatidylserin (PS),
eine Phosphatidsäure,
eine Lysophosphatidsäure, und
mindestens eine an dem PS angelagerte Fettsäure, wobei:

ein Gewichtsprozentsatz des PS bezogen auf das Präparat größer als 40 % ist;
ein Gewichtsprozentsatz von PS bezogen auf die gesamten Phospholipide im Präparat größer als 40 % ist; und
ein Prozentsatz der mindestens einen, an dem PS angelagerten Fettsäure bezogen auf einen gesamten,

an dem PS angelagerten Fettsäuregehalt im Präparat derart ist, dass der Prozentsatz von Eicosapentaensäure (EPA) größer als oder gleich dem Prozentsatz von Palmitinsäure ist, der Prozentsatz von Palmitinsäure größer als der Prozentsatz von Docosahexaensäure (DHA) ist, der Prozentsatz von DHA größer als der Prozentsatz von Oleinsäure ist, und der Prozentsatz von Oleinsäure größer als der Prozentsatz von Linolsäure ist.

**2.** Präparat nach dem vorhergehenden Anspruch, wobei der Gewichtsprozentsatz von PS bezogen auf die gesamten Phospholipide in dem Präparat größer als 50 % ist.

**3.** Präparat nach einem der vorhergehenden Ansprüche, wobei der Prozentsatz von an dem PS angelagerten EPA in dem Präparat bezogen auf den gesamten, an dem PS angelagerten Fettsäuregehalt in dem Präparat größer als 18 % und kleiner als 45 %, gegebenenfalls größer als 27 % und kleiner als 34 % ist.

**4.** Präparat nach einem der vorhergehenden Ansprüche, wobei der Prozentsatz von an dem PS angelagerter Palmitinsäure in dem Präparat bezogen auf den gesamten, an dem PS angelagerten Fettsäuregehalt in dem Präparat größer als 14 % und kleiner als 42 %, gegebenenfalls größer als 21 % und kleiner als 26 % ist.

**5.** Präparat nach einem der vorhergehenden Ansprüche, wobei der Prozentsatz von an dem PS angelagertem DHA in dem Präparat bezogen auf den gesamten, an dem PS angelagerten Fettsäuregehalt in dem Präparat größer als 6 % und kleiner als 25 %, gegebenenfalls größer als 12 % und kleiner als 17 % ist.

**6.** Präparat nach einem der vorhergehenden Ansprüche, wobei der Prozentsatz von an dem PS angelagerter Oleinsäure in dem Präparat bezogen auf den gesamten, an dem PS angelagerten Fettsäuregehalt in dem Präparat größer als 1 % und kleiner als 15 %, gegebenenfalls größer als 5 % und kleiner als 8 % ist.

**7.** Präparat nach einem der vorhergehenden Ansprüche, wobei der Prozentsatz von an dem PS angelagerter Linolsäure in dem Präparat bezogen auf den gesamten, an dem PS angelagerten Fettsäuregehalt in dem Präparat größer als 0,1 % und kleiner als 6,0 %, gegebenenfalls größer als 1 % und kleiner als 2 % ist.

**8.** Präparat nach einem der vorhergehenden Ansprüche, ferner umfassend ein Phosphatidylcholin (PC).

**9.** Präparat nach Anspruch 8, wobei ein Gewichtsprozentsatz des PC bezogen auf das Präparat weniger als 10,0 %, gegebenenfalls größer als 0,01 und kleiner als 3,2 % beträgt.

**10.** Präparat nach einem der vorhergehenden Ansprüche, wobei ein Gewichtsverhältnis des PS zur Summe von Phosphatidsäure und der Lysophosphatidsäure in dem Präparat größer als 1:1 und kleiner als 10:1, gegebenenfalls größer als 2,5:1 und kleiner als 4:1 ist.

**11.** Zusammensetzung, umfassend das Präparat nach einem der vorstehenden Ansprüche, wobei die Zusammensetzung eine Nahrungszusammensetzung, ein Nutrazeutikum, ein funktionelles Lebensmittel oder ein medizinisches Lebensmittel ist.

**12.** Arzneimittel, umfassend das Präparat nach einem der vorstehenden Ansprüche.

**13.** Arzneimittel nach Anspruch 12, ferner umfassend mindestens einen pharmazeutischen Wirkstoff.

**14.** Präparat nach einem der Ansprüche 1-10 oder Zusammensetzung nach den Ansprüchen 11, 12 oder 13 zur Verwendung bei der Behandlung einer Kommunikationsstörung, die einer Störung des autistischen Formenkreises (ASD), Autismus oder dem Asperger-Syndrom zugeordnet ist.

## Revendications

**1.** Préparation comprenant :

une phosphatidyl sérine (PS),
un acide phosphatidique,
un acide lysophosphatidique, et

au moins un acide gras fixé à la PS, dans laquelle :

un pourcentage en poids de la PS par rapport à la préparation est supérieur à 40 % ;
un pourcentage en poids de la PS par rapport aux phospholipides totaux dans la préparation est supérieur à 40 %; et un pourcentage de l'au moins un acide gras fixé à la PS par rapport à la teneur totale en acides gras fixés à la PS dans la préparation est tel que le pourcentage de l'acide eicosa pentaénoïque (EPA) est supérieur ou égal au pourcentage de l'acide palmitique, le pourcentage de l'acide palmitique est supérieur au pourcentage de l'acide docosa hexaénoïque (DHA), le pourcentage de DHA est supérieur au pourcentage de l'acide oléique, et le pourcentage de l'acide oléique est supérieur au pourcentage de l'acide linoléique.

**2.** Préparation selon la revendication précédente, dans laquelle le pourcentage en poids de la PS par rapport aux phospholipides totaux dans la préparation est supérieur à 50 %.

**3.** Préparation selon l'une quelconque des revendications précédentes, dans laquelle le pourcentage de l'EPA fixé à la PS dans la préparation par rapport à la teneur totale en acides gras fixés à la PS dans la préparation est supérieur à 18 % et inférieur à 45 %, éventuellement supérieur à 27 % et inférieur à 34 %.

**4.** Préparation selon l'une quelconque des revendications précédentes, dans laquelle le pourcentage de l'acide palmitique fixé à la PS dans la préparation par rapport à la teneur totale en acides gras fixés à la PS dans la préparation est supérieur à 14 % et inférieur à 42 %, éventuellement supérieur à 21 % et inférieur à 26 %.

**5.** Préparation selon l'une quelconque des revendications précédentes, dans laquelle le pourcentage de DHA fixé à la PS dans la préparation par rapport à la teneur totale en acides gras fixés à la PS dans la préparation est supérieur à 6 % et inférieur à 25 %, éventuellement supérieur à 12 % et inférieur à 17 %.

**6.** Préparation selon l'une quelconque des revendications précédentes, dans laquelle le pourcentage de l'acide oléique fixé à la PS dans la préparation par rapport à la teneur totale en acides gras fixés à la PS dans la préparation est supérieur à 1 % et inférieur à 15 %, éventuellement supérieur à 5 % et inférieur à 8 %.

**7.** Préparation selon l'une quelconque des revendications précédentes, dans laquelle le pourcentage de l'acide linoléique fixé à la PS dans la préparation par rapport à la teneur totale en acides gras fixés à la PS dans la préparation est supérieur à 0,1 % et inférieur à 6,0 %, éventuellement supérieur à 1 % et inférieur à 2 %.

**8.** Préparation selon l'une quelconque des revendications précédentes, comprenant en outre une phosphatidyl choline (PC).

**9.** Préparation selon la revendication 8, dans laquelle un pourcentage en poids de la PC par rapport à la préparation est de moins de 10,0 %, éventuellement supérieur à 0,01 et inférieur à 3,2 %.

**10.** Préparation selon l'une quelconque des revendications précédentes, dans laquelle un ratio en poids entre la PS et la somme de l'acide phosphatidique et de l'acide lysophosphatidique dans la préparation est supérieur à 1 : 1 et inférieur à 10 : 1, éventuellement supérieur à 2,5 : 1 et inférieur à 4 : 1.

**11.** Composition comprenant la préparation selon l'une quelconque des revendications ci-dessus, la composition étant une composition nutritionnelle, nutraceutique, un aliment fonctionnel, ou un aliment médicinal.

**12.** Composition pharmaceutique comprenant la préparation selon l'une quelconque des revendications ci-dessus.

**13.** Composition pharmaceutique selon la revendication 12, comprenant en outre au moins un agent pharmaceutiquement actif.

**14.** Préparation selon l'une quelconque des revendications 1 à 10 ou composition selon les revendications 11, 12 ou 13 destinée à une utilisation dans le traitement de la déficience de la communication associée à un trouble du spectre autistique (TSA), à l'autisme ou au syndrome d'Asperger.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2013289277 A1
- US 2009074857 A1
- WO 2015085192 A2
- WO 2005037848 A
- WO 2009156991 A
- WO 2005038037 A